# EUROPEAN PATENT APPLICATION

(11) **EP 3 862 032 A1**
(43) Date of publication of application: **11.08.2021**
(21) Application number: 21155574.3
(22) Date of filing: 05.02.2021
(51) Int. Cl.: A61L 31/02, A61L 31/10, A61L 31/16, B32B 7/00

(54) **STENTS HAVING BIODEGRADABLE LAYERS**

(30) Priority: 07.02.2020 US 202016784842
(71) Applicant: Micell Technologies, Inc., Durham, NC 27713 (US)
(72) Inventor: MCCLAIN, James B., Ocracoke, NC 27960 (US); TAYLOR, Charles, Douglas, Franklinton, NC 27525 (US); RABINER, Robert, Tiverton, RI 02878 (US)
(74) Representative: Regimbeau

(57) **Abstract**

The invention relates to a laminate coronary stent comprising:
a. a stent framework;
b. a plurality of layers on said stent framework to form said laminate coronary stent, where at least one of said layers comprises a bioabsorbable polymer; and
c. a layer of active agent in crystalline form and an extracellular matrix comprising an interlocking mesh of fibrous proteins and glycosaminoglycans (GAGs).

## Description

### BACKGROUND OF THE INVENTION

The present invention relates to methods for forming stents comprising a bioabsorbable polymer and a pharmaceutical or biological agent in powder form onto a substrate.

It is desirable to have a drug-eluting stent with minimal physical, chemical and therapeutic legacy in the vessel after a proscribed period of time. This period of time is based on the effective healing of the vessel after opening the blockage by PCI/stenting (currently believed by leading clinicians to be 6-18 months).

It is also desirable to have drug-eluting stents of minimal cross-sectional thickness for (a) flexibility of deployment (b) access to small vessels (c) minimized intrusion into the vessel wall and blood.

### SUMMARY OF THE INVENTION

According to the invention, there is provided a laminate coronary stent as defined in claim 1, as well as in the corresponding depending claims.

In one embodiment, the disclosure provides a laminate stent, for instance a laminate coronary stent, comprising:
a. a stent framework;
b. a plurality of layers on said stent framework to form said laminate stent, where at least one of said layers comprises a bioabsorbable polymer; and
c. a layer of active agent in crystalline form and an extracellular matrix comprising an interlocking mesh of fibrous proteins and glycosaminoglycans (GAGs).

In one embodiment, the disclosure provides a method of preparing a laminate coronary stent comprising:
a. providing a stent framework;
b. depositing a plurality of layers on said stent framework to form said laminate coronary stent; wherein at least one of said layers comprises a bioabsorbable polymer. Preferably, the stent framework is bioabsorbable. In one embodiment the stent framework is made of absorbable material comprising magnesium.

One embodiment provides bioabsorbable polymer selected from PGA poly(glycolide), LPLA poly(1-lactide), DLPLA poly(dl-lactide), PCL poly(e-caprolactone) PDO, poly(dioxolane) PGA-TMC, 85/15 DLPLG p(dl-lactide-co-glycolide), 75/25 DLPL, 65/35 DLPLG, 50/50 DLPLG, TMC poly(trimethylcarbonate), p(CPP:SA) poly(1,3-bis-p-(carboxyphenoxy)propane-co-sebacic acid).

In one embodiment, the one or more active agents comprise a macrolide immunosuppressive (limus) drug. Preferably, the macrolide immunosuppressive drug comprises one or more of rapamycin, 40-0-(2-Hydroxyethyl)rapamycin (everolimus), 40-O-Benzyl-rapamycin, 40-0-(4'-Hydroxymethyl)benzyl-rapamycin, 40-O-[4'-(1,2-Dihydroxyethyl)]benzyl-rapamycin, 40-0-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 40-0-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39, 40-O, O-ethylene-rapamycin, (26R)-26-Dihydro-40-O-(2-hydroxy)ethyl-rapamycin, 28-O-Methyl-rapamycin, 4O-O-(2-Aminœthyl)-rapamycin, 4O-O-(2-Acetaminoethyl)-rapamycin 4O-O-(2-Nicotinamidoethyl)-rapamycin, 40-0-(2-(N-Methyl-imidazo-2'-ylcarbethoxamido)ethyl)-rapamycin, 4O-O-(2-Ethoxycarbonylaminoethyl)-rapamycin, 40-0-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus).

In one embodiment, depositing a plurality of layers on said stent framework to form said laminate coronary stent comprises depositing polymer particles on said framework by an RESS process.

In yet another embodiment the disclosure provides a laminate coronary stent comprising
a. a stent framework;
b. a plurality of layers deposited on said stent framework to form said laminate coronary stent; wherein at least one of said layers comprises a bioabsorbable polymer.

Yet another embodiment provides a method of preparing a laminate coronary stent comprising:
a. providing a stent framework;
b. depositing a plurality of layers on said stent framework to form said laminate coronary stent; wherein at least one of said layers comprises a bioabsorbable polymer; at least one pharmaceutical agent in a therapeutically desirable morphology and/or at least one active biological agent; wherein depositing each layer of said plurality of layers on said stent framework comprises the following steps:
   i. forming a supercritical or near supercritical fluid solution comprising at least one supercritical fluid solvent and one or more pharmaceutical agents and/or at least one active biological agent discharging said supercritical or near supercritical fluid solution through a first orifice under conditions sufficient to form solid particles of said one or more pharmaceutical agents and/or at least one active biological agent;
   ii. forming a supercritical or near supercritical fluid solution comprising at least one supercritical fluid solvent and at least one polymer and discharging said supercritical or near supercritical fluid solution through said first orifice or through a second orifice under conditions sufficient to form solid particles of the polymer;
   iii. depositing the polymer and pharmaceutical agent and/or active biological agent particles onto said framework, wherein an electrical potential is maintained between the framework and the polymer and pharmaceutical agent and/or active biological agent particles, thereby forming said layer; and
   iv. sintering said layer under conditions that do not substantially modify the morphology of said pharmaceutical agent and/or the activity of said biological agent. Preferably, the framework is electrostatically charged. In one embodiment, framework is biodegradable.

Yet another embodiment, provides a method of preparing coronary stent comprising:
a. forming a sheet comprising a bioabsorbable polymer;
b. carving out a pattern of said coronary stent into said sheet; and
c. rolling said sheet to form said coronary stent. In one embodiment, forming said sheet comprises depositing a plurality of layers to form said sheet and said coronary stent is a laminate coronary stent.

### INCORPORATION BY REFERENCE

All publications and patent applications mentioned in this specification are herein incorporated by reference to the same extent as if each individual publication or patent application was specifically and individually indicated to be incorporated by reference.

### BRIEF DESCRIPTION OF THE DRAWINGS

Figures 1-10 illustrate various embodiments of the invention.
Figure 1 shows a cross-section of a single strut including laminated layers of bioabsorbable polymer and drugs.
Figure 2 shows polymer nanoparticles fused via SCF with no solvents or high temperatures. The final material thus provides a smooth, adherently laminated layer with precise control over location of drug(s).
Figure 3 is a graphical representation of the percentage of elution over time.
Figure 4 shows mechanically effective coating with and without parylene base-coat.
Figure 5 shows stents coating and sintered resulting in a conformal and even film over all aspects of the device. Thus, demonstrating in a single layer for: smooth conformal and mechanically adherent coating on different stent substrates; wide range of drugs (rapamycin, paclitaxel, heparin, small molecules, etc.); and multiple and dissimilar drugs (paclitaxel + heparin) in the same coating.
Figure 6 shows rapamycin DES coating with drug maintained in crystalline morphology. Thus, in a single layer for: control over drug morphology: crystalline or amorphous; maintain drug stability; and no effect on elution vs. commercial analogs. The graphic representation in Figure 6 shows the peak area ration between the control samples (left two bars) and the Micell processed materials (right two bars) indicating no difference in the rate of rapamycin degradation.
Figure 7 shows a thin, conformal, defect-free coating at target thicknesses.
Figure 8 shows controlled drug placement with the Confocal Raman spectra on the left and side showing drug loaded purposefully in the center of the 10 µm DES coating. The SIMS of DES coating surface shown in the right side of Figure 8 shows the drug loaded equally throughout the 10 µm DES coating and evident in the surface.
Figure 9 shows current coatings and stents are unable to dial-in drugs' location and elution. The graphical representation shown in Figure 9 shows elution data for heparin + taxol in DES coating with the addition of a 2nd drug (heparin) not affecting primary drug (taxol) elution profile, and with the heparin elution continuing after 15 days [preliminary data].
Figure 10 shows multi-drug elution. The "ideal" stent is one in which the drug and polymer disappear simultaneously - Dr. Alan Yeung, Director of Interventional Cardiology, Stanford University Medical Center.

### DETAILED DESCRIPTION OF THE INVENTION

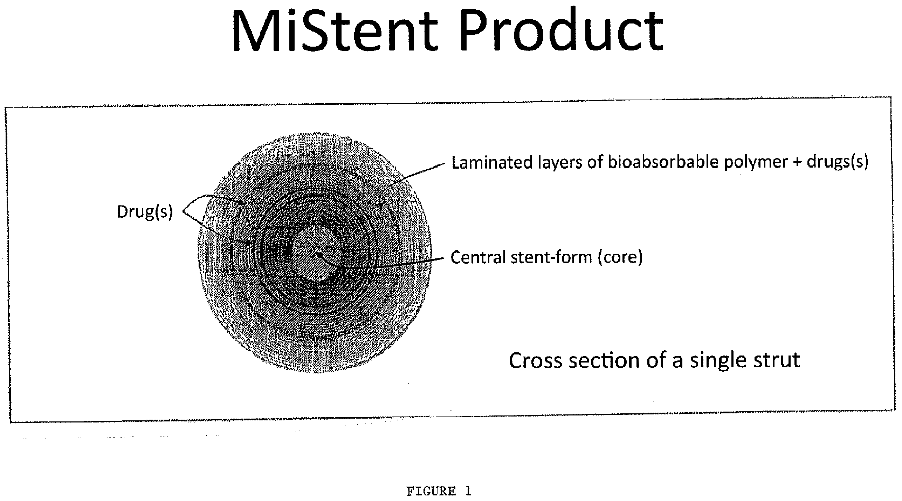

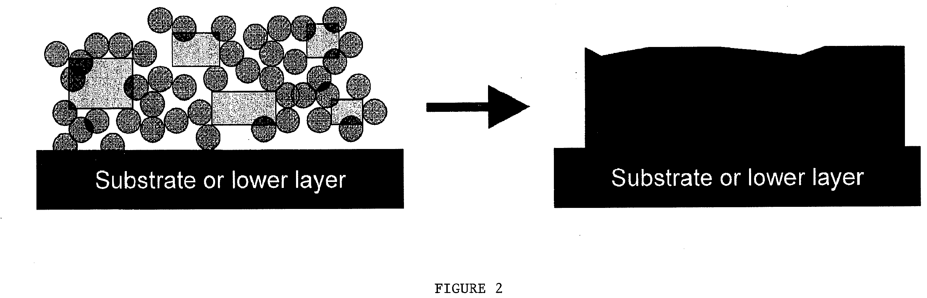

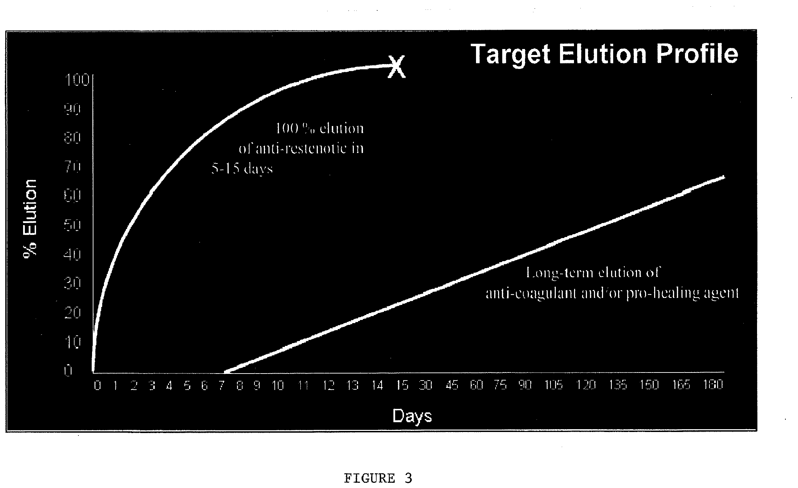

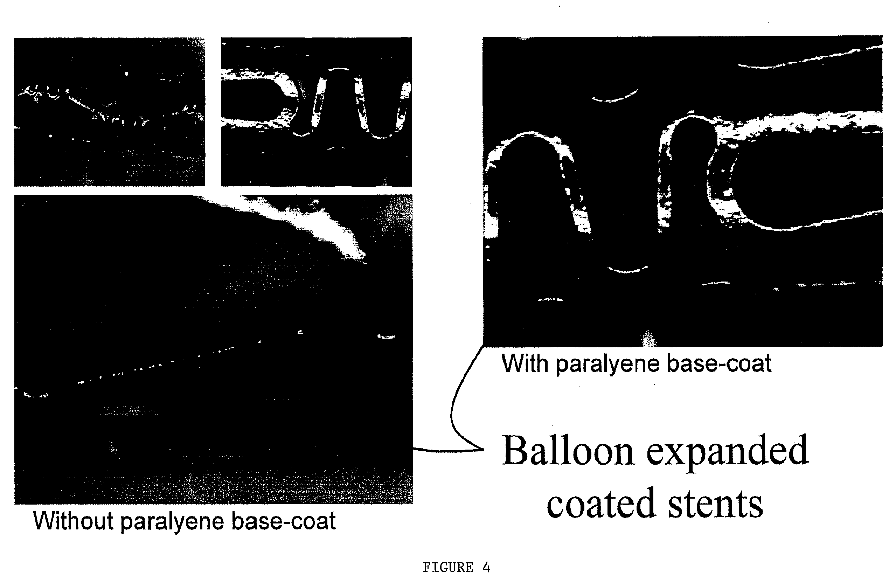

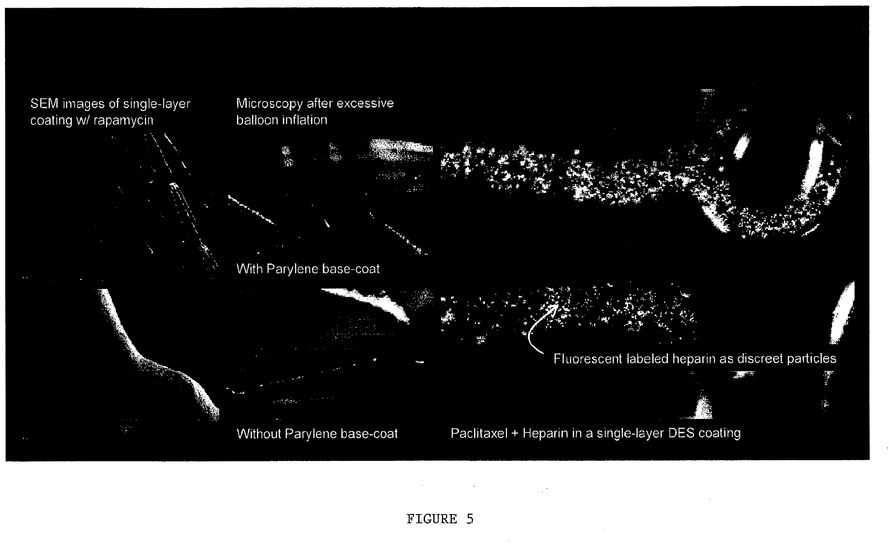

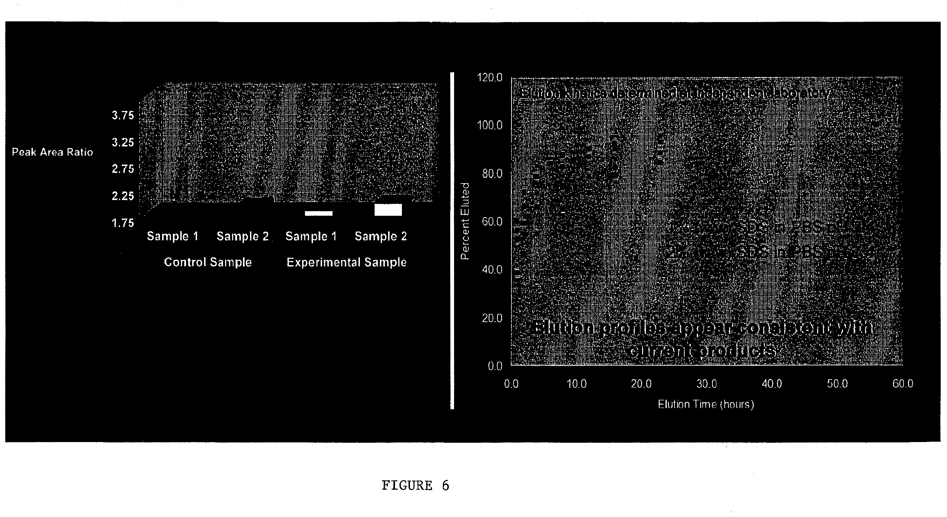

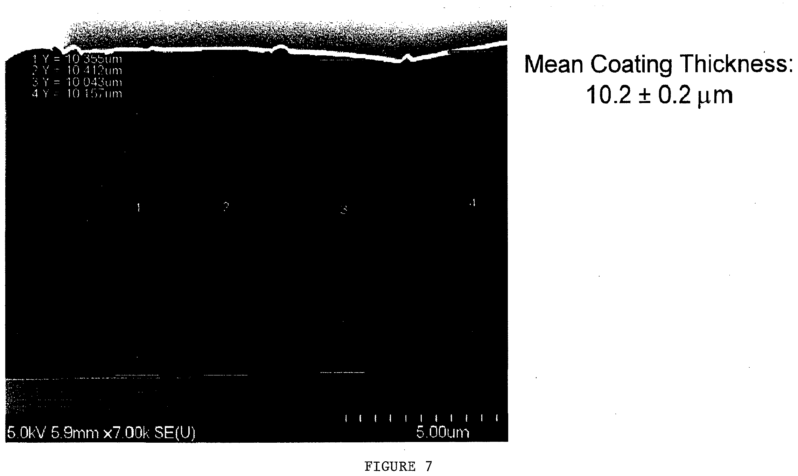

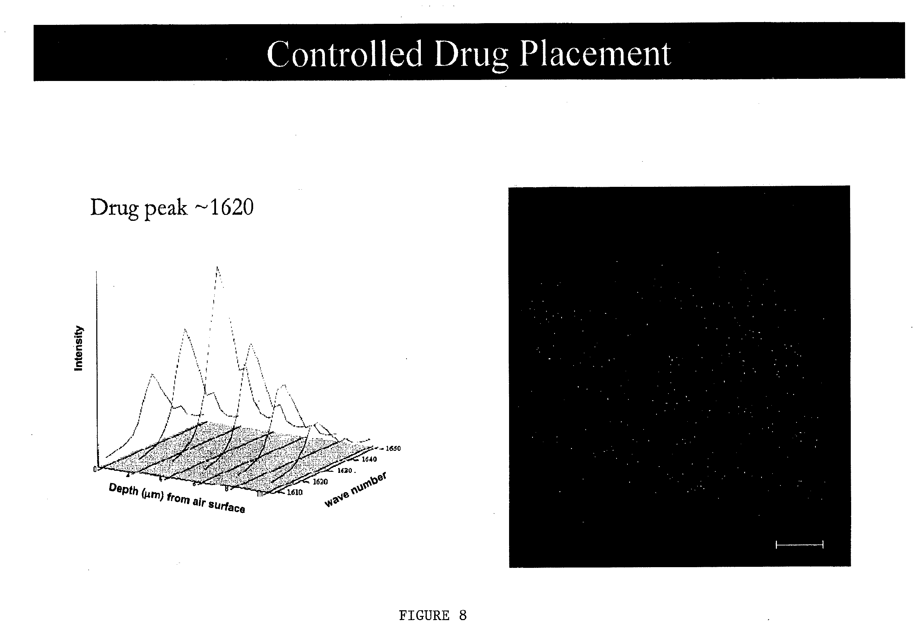

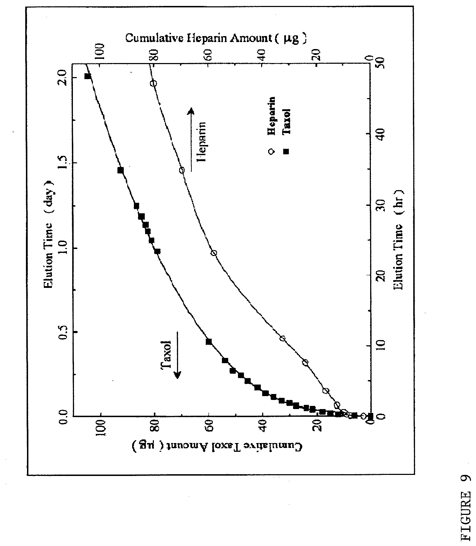

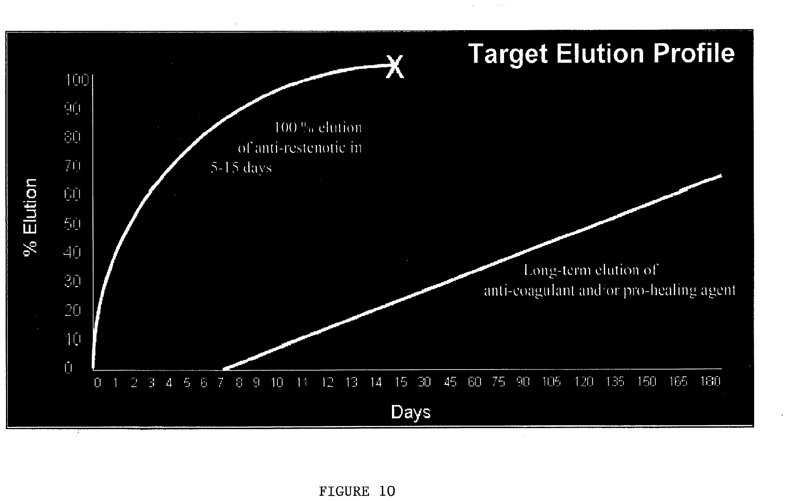

Illustration of selected embodiments of the inventions is provided in appended Figures 1-10.

The present invention is explained in greater detail below. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. In addition, numerous variations and additions to the various embodiments suggested herein will be apparent to those skilled in the art in light of the instant disclosure, which do not depart from the instant invention. Hence, the following specification is intended to illustrate some particular embodiments of the invention, and not to exhaustively specify all permutations, combinations and variations thereof.

Applicants specifically intend that all United States patent references cited herein be incorporated herein by reference in their entirety.

In one embodiment, the invention provides a process wherein a stent is constructed from the bottom-up. A stent-form (or framework) will be used as a template, onto which a laminated structure of bioabsorbable polymer(s) + drug(s) is overlaid - forming the final stent. This final stent may be fully bioabsorbable if an absorbable stent-form is utilized. In the case of a non-absorbable stent-form (e.g. stainless steel), all of the polymer and drug shall be absorbed/eluted, leaving only the very thin metallic stent-form embedded in the vessel wall.

In one embodiment, the stent-form can be an exact structural replica of the to-be-produced stent. In this embodiment, the stent form would have approximately the same longitudinal and radial dimensions as the final stent. The stent form would have between 2x and 100x thinner wires/struts.

The laminated structure provides significantly improved mechanical properties in a predominantly polymer-based, bioabsorbable, balloon-expandable stent. The laminated structure that results from successive coatings on the stent-form provides a mechanically effective stent (e.g. with deployed hoop strength > 300mm Hg luminal pressure) with thinner struts than traditional metallic stents and which are superior to the known bioabsorbable polymeric stents.

In creating the bioabsorbable stent, the present methods apply several layers (2-100) of bioabsorbable polymer(s) to the stent form (coat-sinter-coat-sinter, repeat). This will result in a laminated polymer structure upon the stent-form, thus building the struts up to the target dimensions for use (which may be smaller, the same or larger than a metallic stent of similar strut design - depending on the desired mechanical properties of the stent). Single or multiple drugs may be included in one, some or all of these layers. Alternatively the drugs could be located substantially between the polymer layers.

This discreet location of drug/drugs is designed to provide specific, time-targeted elution profiles and may enable the elution of multiple drugs in serial fashion.

Elements of this embodiment include:
A. The stent form:
   - Create a stent-form directly via stent manufacturing processes (e.g. laser cutting of tube stock, lithographic transfer to sheet stock, etc.). The stent-form of the present invention is 2-100x thinner than a traditional stent (which makes it, in-and-of-itself, difficult to use without the added strength and physical properties of the polymer laminate(s)).
   - Create a stent-form by etching an existing permanent metal stent (stainless steel, Cobalt-Chromium, Nitinol, platinum alloy, gold, silver, etc.) to create the wire frame in the template of the original stent.Create a stent-form from a bioadsorbable conductive material to use as a template for the subsequently applied biodegradable polymer(s) + drug(s) (e.g. conductive polymeric materials or magnesium as described in: J Interv Cardiol. 2004 December 17(6):391-5.
B. Means for creating the bioabsorbable polymer(s) + drug (s) matrix on the stent-form - forming the final device.
   - Spray coat the stent-form with drug and polymer as is done in Micell process (e-RESS, e-DPC, compressed-gas sintering).
   - Perform multiple and sequential coating-sintering steps where different materials may be deposited in each step, thus creating a laminated structure with a multitude of thin layers of drug(s), polymer(s) or drug+polymer that build the final stent.
   - Perform the deposition of polymer(s) + drug(s) laminates with the inclusion of a mask on the inner (luminal) surface of the stent. Such a mask could be as simple as a non-conductive mandrel inserted through the internal diameter of the stent form. This masking could take place prior to any layers being added, or be purposefully inserted after several layers are deposited continuously around the entire stent-form.

In another embodiment, the invention provides a process wherein the pattern of a stent is carved out of a sheet of polymeric material (e.g., a flat sheet). In one embodiment, the polymeric sheet is a bioabsorbable polymer + drug(s) formulation. Further, the sheet may contain a laminated structure of multiple layers (2-100) made from one or more bioabsorbable polymers. The sheet may contain none, one or multiple drugs. After the pattern of the stent is carved into the polymer sheet, the sheet is rolled into the specified diameter for the stent. This rolled stent is welded into a cylindrical form.

The laminated structure provides significantly improved mechanical properties in a predominantly polymer-based, bioabsorbable, balloon-expandable stent. The laminated structure that results from successive coatings on the stent-form may provide a mechanically effective stent (e.g. with deployed hoop strength > 300mm Hg luminal pressure) with thinner struts than traditional metallic stents and certainly superior to the known bioabsorbable polymeric stents.

This discreet location of drug/drugs is designed to provide specific, time-targeted elution profiles and may enable the elution of multiple drugs in serial fashion.

The Polymer Laminate: The polymeric sheet material is ideally comprised of bioabsorbable polymers. The polymeric sheet material is ideally a laminated structure. In the laminate, we envision ideal properties to be obtained by alternating 'hard'-'soft' bioabsorbable polymers. The first material aspect of the invention is the creation and unique properties of the polymer film.

Forming the polymer sheet:
- Film-forming processes (spin coating, solvent casting, extrusion, blow-molding
- Micell's compressed-gas methods of eRESS, eDPC and Sintering. By successive coating of a sheet substrate, the polymer sheet could be formed by a succession of coat-sinter-coat-sinter-coat-sinter steps.

Inclusion of drug: The drug can be formulated in one of three places within the polymer sheet.

Upon the surface of the material (either luminal or ab-luminal). Note that such a surface treatment of drug could be applied at any step in the stent manufacturing process: sheet, carved, rolled, welded, final.

Within one of the polymer films comprising the laminate.

Between two of the polymer films comprising the laminate.

Carving of the stent architecture: Methods for the carving include, without limitation: physical cutting by press, roller, knife, jet, or laser and/or chemically etched by wet-chemistry or dry-plasma means. One alternative method of carving may be to physically carve the stent architecture while the polymer sheet is exposed to a compressed fluid environment (e.g. similar to Micell's sintering process). Such exposure could 'soften' the polymer sheet aiding in carving.

All manner of current polymer sheet-forming technologies would apply; including the ancillary necessities of lubricants, mold-release, etc.

Rolling and welding of the carved sheet into a final stent: The final step in the process is to roll the carved sheet into a cylinder and weld this geometry in place. Rolling is straightforward processing around a sized mandrel (may be augmented by temperature or exposure to compressed fluids). The welding may be performed via conventional methods such as solvent, temperature (bulk heating, laser, etc.), etc.. The welding process is preferably done via compressed-fluid-based sintering.

The invention provides improved mechanical properties compared to conventional bioabsorbable stents. The present invention provides a laminated bioabsorbable polymer stent, with significantly increased in strength, deformability, hoop stress (both pre- and post-expansion) and other mechanical properties. A laminate structure is inherently stronger and more effective in the deformation processes necessary for DES use (crimping onto a balloon, expansion into the diseased vessel). Also the invention provides the ability to obtain greater hoop-strength in the deployed stent based on the laminate architecture of the stents. For example, the present invention allows the formation of a laminate of different bioabsorbable polymers (e.g., PLA, PGA and copolymers thereof) with different mechanical properties: hard-soft-hard-soft-hard-soft type of laminated structure.

One attribute of a hard-soft structure will be to provide a unique and novel control of the pressure needed for expansion of the stent. By designing the soft laminate layer to act as the 'slip layer' or 'lubrication' between neighboring layers the stress needed for expansion can be 'dialed in' (range 3-30 atm pressure for full expansion).

Another advantage of the present invention is the ability to create a stent with a completely novel drug-elution profile. Via the ability to have different materials in each layer of the laminate structure and the ability to control the location of drug(s) independently in these layers, the method enables a bioabsorbable stent that could release drugs at very specific elution profiles, programmed sequential and/or parallel elution profiles. Also, the present invention allows controlled elution of one drug without affecting the elution of a second drug (or different doses of the same drug).

The embodiments incorporating a stent form or framework provide the ability to radiographically monitor the stent in deployment. In an alternative embodiment, the inner-diameter of the stent can be masked (e.g. by a non-conductive mandrel). Such masking would prevent additional layers from being on the interior diameter (abluminal) surface of the stent. The resulting configuration may be desirable to provide preferential elution of the drug toward the vessel wall (luminal surface of the stent) where the therapeutic effect of anti-restenosis is desired, without providing the same antiproliferative drug(s) on the abluminal surface, where they may retard healing, which in turn is suspected to be a cause of late-stage safety problems with current DESs.

The present invention provides numerous advantages. The invention is advantageous allows for employing a platform combining layer formation methods based on compressed fluid technologies; electrostatic capture and sintering methods. The platform results in drug eluting stents having enhanced therapeutic and mechanical properties. The invention is particularly advantageous in that it employs optimized laminate polymer technology. In particular, the present invention allows the formation of discrete layers of specific drug platforms.

Conventional processes for spray coating stents require that drug and polymer be dissolved in solvent or mutual solvent before spray coating can occur. The platform provided herein the drugs and polymers are coated on the stent framework in discrete steps, which can be carried out simultaneously or alternately. This allows discrete of the active agent (e.g.; a drug) within a polymer matrix thereby allowing the placement of more than one drug on a single active agent on a single medical device with or without an intervening polymer layer. For example, the present platform provides a dual drug eluting stent.

Some of the advantages provided by the subject invention include employing compressed fluids (e.g., supercritical fluids, for example E-RESS based methods); solvent free deposition methodology; a platform that allows processing at lower temperatures thereby preserving the qualities of the active agent and the polymer matrix; the ability to incorporate two, three or more drugs while minimizing deleterious effects from direct interactions between the various drugs and/or their excipients during the fabrication and/or storage of the drug eluting stents; a dry deposition; enhanced adhesion and mechanical properties of the layers on the stent framework; precision deposition and rapid batch processing; and ability to form intricate structure.

In one embodiment, the present invention provides a multi-drug delivery platform which produces strong, resilient and flexible drug eluting stents including an anti-restenosis drug (e.g.; a limus or taxol) and anti-thrombosis drug (e.g.; heparin or an analog thereof) and well characterized bioabsorbable polymers. The drug eluting stents provided herein minimize potential for thrombosis, in part, by reducing or totally eliminating thrombogenic polymers and reducing or totally eliminating residual drugs that could inhibit healing.

The platform provides optimized delivery of multiple drug therapies for example for early-stage treatment (restenosis) and late-stage (thrombosis).

The platform also provides an adherent coating which enables access through tortuous lesions without the risk of the coating being compromised.

Another advantage of the present platform is the ability to provide highly desirable eluting profiles (e.g., the profile illustrated in Figures 1-10).

Advantages of the invention include the ability to reduce or completely eliminate potentially thrombogenic polymers as well as possibly residual drugs that may inhibit long term healing. As well, the invention provides advantageous stents having optimized strength and resilience if coatings which in turn allows access to complex lesions and reduces or completely eliminates delamination. Laminated layers of bioabsorbable polymers allow controlled elution of one or more drugs.

The platform provided herein reduces or completely eliminates shortcoming that have been associated with conventional drug eluting stents. For example, the platform provided herein allows for much better tuning of the period of time for the active agent to elute and the period of time necessary for the polymer matrix to resorb thereby minimizing thrombosis and other deleterious effects associate with poorly controlled drug release.

Additional advantages of the present platform are illustrated in Figures 1-10

### Definitions

As used in the present specification, the following words and phrases are generally intended to have the meanings as set forth below, except to the extent that the context in which they are used indicates otherwise.

"Substrate" as used herein, refers to any surface upon which it is desirable to deposit a coating comprising a polymer and a pharmaceutical or biological agent, wherein the coating process does not substantially modify the morphology of the pharmaceutical agent or the activity of the biological agent. Biomedical implants are of particular interest for the present invention; however the present invention is not intended to be restricted to this class of substrates. Those of skill in the art will appreciate alternate substrates that could benefit from the coating process described herein, such as pharmaceutical tablet cores, as part of an assay apparatus or as components in a diagnostic kit (e.g. a test strip).

"Biomedical implant" as used herein refers to any implant for insertion into the body of a human or animal subject, including but not limited to stents (e.g., vascular stents), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, etc.

The implants may be formed from any suitable material, including but not limited to organic polymers (including stable or inert polymers and biodegradable polymers), metals, inorganic materials such as silicon, and composites thereof, including layered structures with a core of one material and one or more coatings of a different material. Substrates made of a conducting material facilitate electrostatic capture. However, the invention contemplates the use of electrostatic capture in conjunction with substrate having low conductivity or which non-conductive. To enhance electrostatic capture when a non-conductive substrate is employed, the substrate is processed while maintaining a strong electrical field in the vicinity of the substrate.

Subjects into which biomedical implants of the invention may be applied or inserted include both human subjects (including male and female subjects and infant, juvenile, adolescent, adult and geriatric subjects) as well as animal subjects (including but not limited to dog, cat, horse, monkey, etc.) for veterinary purposes.

In a preferred embodiment the biomedical implant is an expandable intraluminal vascular graft or stent (e.g., comprising a wire mesh tube) that can be expanded within a blood vessel by an angioplasty balloon associated with a catheter to dilate and expand the lumen of a blood vessel, such as described in US Patent No. 4,733,665 to Palmaz Shaz.

"Pharmaceutical agent" as used herein refers to any of a variety of drugs or pharmaceutical compounds that can be used as active agents to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the pharmaceutical agents of the invention may also comprise two or more drugs or pharmaceutical compounds. Pharmaceutical agents, include but are not limited to antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, antigens, beta-receptor blockers, non-steroidal antiinflammatory drugs {NSAIDs], cardiac glycosides, acetylsalicylic acid, virustatics, aclarubicin, acyclovir, cisplatin, actinomycin, alpha- and beta-sympatomimetics, (dmeprazole, allopurinol, alprostadil, prostaglandins, amantadine, ambroxol, amlodipine, methotrexate, S-aminosalicylic acid, amitriptyline, amoxicillin, anastrozole, atenolol, azathioprine, balsalazide, beclomethasone, betahistine, bezafibrate, bicalutamide, diazepam and diazepam derivatives, budesonide, bufexamac, buprenorphine, methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidine, cotrimoxazole, codeine, caffeine, vitamin D and derivatives of vitamin D, colestyramine, cromoglicic acid, coumarin and coumarin derivatives, cysteine, cytarabine, cyclophosphamide, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxorubizin, doxylamine, dapiprazole, benzodiazepines, diclofenac, glycoside antibiotics, desipramine, econazole, ACE inhibitors, enalapril, ephedrine, epinephrine, epoetin and epoetin derivatives, morphinans, calcium antagonists, irinotecan, modafinil, orlistat, peptide antibiotics, phenytoin, riluzoles, risedronate, sildenafil, topiramate, macrolide antibiotics, oestrogen and oestrogen derivatives, progestogen and progestogen derivatives, testosterone and testosterone derivatives, androgen and androgen derivatives, ethenzamide, etofenamate, etofibrate, fenofibrate, etofylline, etoposide, famciclovir, famotidine, felodipine, fenofibrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tetracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobuterol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristine, vindesine, vinorelbine, vinpocetine, viquidil, warfarin, xantinol nicotinate, xipamide, zafirlukast, zalcitabine, zidovudine, zolmitriptan, zolpidem, zoplicone, zotipine and the like. See, e.g., US Patent No. 6,897,205; see also US Patent No. 6,838,528; US Patent No. 6,497,729.

Examples of therapeutic agents employed in conjunction with the invention include, rapamycin, 40-0-(2-Hydroxyethyl)rapamycin (everolimus), 40-0-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 40-0-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus).

The active ingredients may, if desired, also be used in the form of their pharmaceutically acceptable salts or derivatives (meaning salts which retain the biological effectiveness and properties of the compounds of this invention and which are not biologically or otherwise undesirable), and in the case of chiral active ingredients it is possible to employ both optically active isomers and racemates or mixtures of diastereoisomers.

The active ingredients can also comprise at least one extracellular matrix and an extracellular matrix component. The extracellular matrix component can comprise at least one of: heparin sulfate, chondroitin sulfate, keratan sulfate, hyaluronic acid, collagen, elastin, fibronectin, laminin, merosin, tenascin, vitronectin, and fibrillin. The extracellular matrix includes the interstitial matrix and the basement membrane. Interstitial matrix is present between various animal cells (i.e., the intercellular spaces). Gels of polysaccharides and fibrous proteins fill the interstitial space and act as a compression buffer against the stress placed on the ECM. The ECM is composed of an interlocking mesh of fibrous proteins and glycosaminoglycans (GAGs).

"Stability" as used herein in refers to the stability of the drug in a polymer coating deposited on a substrate in its final product form (e.g., stability of the drug in a coated stent). The term stability will define 5% or less degradation of the drug in the final product form.

"Active biological agent" as used herein refers to a substance, originally produced by living organisms, that can be used to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the active biological agents of the invention may also comprise two or more active biological agents or an active biological agent combined with a pharmaceutical agent, a stabilizing agent or chemical or biological entity. Although the active biological agent may have been originally produced by living organisms, those of the present invention may also have been synthetically prepared, or by methods combining biological isolation and synthetic modification. By way of a non-limiting example, a nucleic acid could be isolated form from a biological source, or prepared by traditional techniques, known to those skilled in the art of nucleic acid synthesis. Furthermore, the nucleic acid may be further modified to contain non-naturally occurring moieties. Non-limiting examples of active biological agents include peptides, proteins, enzymes, glycoproteins, nucleic acids (including deoxyribonucleotide or ribonucleotide polymers in either single or double stranded form, and unless otherwise limited, encompasses known analogues of natural nucleotides that hybridize to nucleic acids in a manner similar to naturally occurring nucleotides), antisense nucleic acids, fatty acids, antimicrobials, vitamins, hormones, steroids, lipids, polysaccharides, carbohydrates and the like. They further include, but are not limited to, antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals and chemotherapeutic agents. Preferably, the active biological agent is a peptide, protein or enzyme, including derivatives and analogs of natural peptides, proteins and enzymes.

"Activity" as used herein refers to the ability of a pharmaceutical or active biological agent to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). Thus the activity of a pharmaceutical or active biological agent should be of therapeutic or prophylactic value.

"Secondary, tertiary and quaternary structure " as used herein are defined as follows. The active biological agents of the present invention will typically possess some degree of secondary, tertiary and/or quaternary structure, upon which the activity of the agent depends. As an illustrative, non-limiting example, proteins possess secondary, tertiary and quaternary structure. Secondary structure refers to the spatial arrangement of amino acid residues that are near one another in the linear sequence. The α-helix and the β-strand are elements of secondary structure. Tertiary structure refers to the spatial arrangement of amino acid residues that are far apart in the linear sequence and to the pattern of disulfide bonds. Proteins containing more than one polypeptide chain exhibit an additional level of structural organization. Each polypeptide chain in such a protein is called a subunit. Quaternary structure refers to the spatial arrangement of subunits and the nature of their contacts. For example hemoglobin consists of two α and two β chains. It is well known that protein function arises from its conformation or three dimensional arrangement of atoms (a stretched out polypeptide chain is devoid of activity). Thus one aspect of the present invention is to manipulate active biological agents, while being careful to maintain their conformation, so as not to lose their therapeutic activity.

"Polymer" as used herein, refers to a series of repeating monomeric units that have been cross-linked or polymerized. Any suitable polymer can be used to carry out the present invention. It is possible that the polymers of the invention may also comprise two, three, four or more different polymers. In some embodiments, of the invention only one polymer is used. In some preferred embodiments a combination of two polymers are used. Combinations of polymers can be in varying ratios, to provide coatings with differing properties. Those of skill in the art of polymer chemistry will be familiar with the different properties of polymeric compounds.

"Therapeutically desirable morphology" as used herein refers to the gross form and structure of the pharmaceutical agent, once deposited on the substrate, so as to provide for optimal conditions of ex vivo storage, in vivo preservation and/or in vivo release. Such optimal conditions may include, but are not limited to increased shelf life, increased in vivo stability, good biocompatibility, good bioavailability or modified release rates. Typically, for the present invention, the desired morphology of a pharmaceutical agent would be crystalline or semi-crystalline or amorphous, although this may vary widely depending on many factors including, but not limited to, the nature of the pharmaceutical agent, the disease to be treated/prevented, the intended storage conditions for the substrate prior to use or the location within the body of any biomedical implant. Preferably at least 10%, 20%, 30%, 40%, 50%, 60%, 70%, 80%, 90% or 100% of the pharmaceutical agent is in crystalline or semi-crystalline form.

"Stabilizing agent" as used herein refers to any substance that maintains or enhances the stability of the biological agent. Ideally these stabilizing agents are classified as Generally Regarded As Safe (GRAS) materials by the US Food and Drug Administration (FDA). Examples of stabilizing agents include, but are not limited to carrier proteins, such as albumin, gelatin, metals or inorganic salts. Pharmaceutically acceptable excipient that may be present can further be found in the relevant literature, for example in the Handbook of Pharmaceutical Additives: An International Guide to More Than 6000 Products by Trade Name, Chemical, Function, and Manufacturer; Michael and Irene Ash (Eds.); Gower Publishing Ltd.; Aldershot, Hampshire, England, 1995.

"Compressed fluid" as used herein refers to a fluid of appreciable density (e.g., >0.2 g/cc) that is a gas at standard temperature and pressure. "Supercritical fluid", "near-critical fluid", "near-supercritical fluid", "critical fluid", "densified fluid" or "densified gas" as used herein refers to a compressed fluid under conditions wherein the temperature is at least 80% of the critical temperature of the fluid and the pressure is at least 50% of the critical pressure of the fluid.

Examples of substances that demonstrate supercritical or near critical behavior suitable for the present invention include, but are not limited to carbon dioxide, isobutylene, ammonia, water, methanol, ethanol, ethane, propane, butane, pentane, dimethyl ether, xenon, sulfur hexafluoride, halogenated and partially halogenated materials such as chlorofluorocarbons, hydrochlorofluorocarbons, hydrofluorocarbons, perfluorocarbons (such as perfluoromethane and perfuoropropane, chloroform, trichloro-fluoromethane, dichloro-difluoromethane, dichloro-tetrafluoroethane) and mixtures thereof.

"Sintering" as used herein refers to the process by which parts of the matrix or the entire polymer matrix becomes continuous (e.g., formation of a continuous polymer film). As discussed below, the sintering process is controlled to produce a fully conformal continuous matrix (complete sintering) or to produce regions or domains of continuous coating while producing voids (discontinuities) in the matrix. As well, the sintering process is controlled such that some phase separation is obtained between polymer different polymers (e.g., polymers A and B) and/or to produce phase separation between discrete polymer particles. Through the sintering process, the adhesions properties of the coating are improved to reduce flaking of detachment of the coating from the substrate during manipulation in use. As described below, in some embodiments, the sintering process is controlled to provide incomplete sintering of the polymer matrix. In embodiments involving incomplete sintering, a polymer matrix is formed with continuous domains, and voids, gaps, cavities, pores, channels or, interstices that provide space for sequestering a therapeutic agent which is released under controlled conditions. Depending on the nature of the polymer, the size of polymer particles and/or other polymer properties, a compressed gas, a densified gas, a near critical fluid or a super-critical fluid may be employed. In one example, carbon dioxide is used to treat a substrate that has been coated with a polymer and a drug, using dry powder and RESS electrostatic coating processes. In another example, isobutylene is employed in the sintering process. In other examples a mixture of carbon dioxide and isobutylene is employed.

When an amorphous material is heated to a temperature above its glass transition temperature, or when a crystalline material is heated to a temperature above a phase transition temperature, the molecules comprising the material are more mobile, which in turn means that they are more active and thus more prone to reactions such as oxidation. However, when an amorphous material is maintained at a temperature below its glass transition temperature, its molecules are substantially immobilized and thus less prone to reactions. Likewise, when a crystalline material is maintained at a temperature below its phase transition temperature, its molecules are substantially immobilized and thus less prone to reactions. Accordingly, processing drug components at mild conditions, such as the deposition and sintering conditions described herein, minimizes cross-reactions and degradation of the drug component. One type of reaction that is minimized by the processes of the invention relates to the ability to avoid conventional solvents which in turn minimizes autoxidation of drug, whether in amorphous, semi-crystalline, or crystalline form, by reducing exposure thereof to free radicals, residual solvents and autoxidation initiators.

"Rapid Expansion of Supercritical Solutions" or "RESS" as used herein involves the dissolution of a polymer into a compressed fluid, typically a supercritical fluid, followed by rapid expansion into a chamber at lower pressure, typically near atmospheric conditions. The rapid expansion of the supercritical fluid solution through a small opening, with its accompanying decrease in density, reduces the dissolution capacity of the fluid and results in the nucleation and growth of polymer particles. The atmosphere of the chamber is maintained in an electrically neutral state by maintaining an isolating "cloud" of gas in the chamber. Carbon dioxide or other appropriate gas is employed to prevent electrical charge is transferred from the substrate to the surrounding environment.

"Bulk properties" properties of a coating including a pharmaceutical or a biological agent that can be enhanced through the methods of the invention include for example: adhesion, smoothness, conformality, thickness, and compositional mixing.

"Electrostatically charged" or "electrical potential" or "electrostatic capture" as used herein refers to the collection of the spray-produced particles upon a substrate that has a different electrostatic potential than the sprayed particles. Thus, the substrate is at an attractive electronic potential with respect to the particles exiting, which results in the capture of the particles upon the substrate. i.e. the substrate and particles are oppositely charged, and the particles transport through the fluid medium of the capture vessel onto the surface of the substrate is enhanced via electrostatic attraction. This may be achieved by charging the particles and grounding the substrate or conversely charging the substrate and grounding the particles, or by some other process, which would be easily envisaged by one of skill in the art of electrostatic capture.

The present invention provides several advantages which overcome or attenuate the limitations of current technology for bioabsorbable stents. Fro example, an inherent limitation of conventional bioabsorbable polymeric materials relates to the difficulty in forming to a strong, flexible, deformable (e.g. balloon deployable) stent with low profile. The polymers generally lack the strength of high-performance metals. The present invention overcomes these limitations by creating a laminate structure in the essentially polymeric stent. Without wishing to be bound by any specific theory or analogy, the increased strength provided by the stents of the invention can be understood by comparing the strength of plywood vs. the strength of a thin sheet of wood.

Embodiments of the invention involving a thin metallic stent-framework provide advantages including the ability to overcome the inherent elasticity of most polymers. It is generally difficult to obtain a high rate (e.g., 100%) of plastic deformation in polymers (compared to elastic deformation where the materials have some 'spring back' to the original shape). Again, without wishing to be bound by any theory, the central metal stent framework (that would be too small and weak to serve as a stent itself) would act like wires inside of a plastic, deformable stent, basically overcoming any 'elastic memory' of the polymer.

### Examples

The following examples are given to enable those skilled in the art to more clearly understand and to practice the present invention. They should not be considered as limiting the scope of the invention, but merely as being illustrative and representative thereof.

### Example 1.

In this example a sheet of polymer film is imprinted by rolling a cylinder across the surface of the sheet. The polymer sheet is made from bioabsorbable polymers prepared by spraying alternating layers of different polymers onto a conductive substrate. The order in which the layers are applied is determined by the desired film mechanical properties. Drug may be applied between each layer or selectively between desired layers. Drug is applied using a dry powder coating technique. A cylindrical, patterned rod is rolled across the polymer film creating a stent in a gravure printing like process. Several methods exist for creating the pattered rod such as using photoresist-etch process. Alternatively the pattern could be laser cut into the solid rod. The flat polymer sheet is cut into strips with widths slightly greater than the circumference of the finished stent. The polymer strips are then rolled around a lubricious non-patterned cylinder (i.e., Teflon) that acts as a form. This object is then placed in pressure vessel and sealed. Gas such as dichlorofluoromethane is added to the pressure vessel until the pressure inside the vessel equals the vapor pressure of the gas at the temperature of the vessel. A suitable gas is dichlorofluoromethane at a temperature of 37 °C. The gas sinters the polymer strip and welds the seam together creating a polymeric stent supported on a Teflon cylinder. After the stent is sintered it is slid off the support.

### Example 2.

In this example a bioabsorable metal such as magnesium is used as a form onto which bioabsorbable polymer(s) can be sprayed in layered fashion. A polymer layer of one type such as PLA is sprayed on the stent and sintered. A second polymer layer of another type such as PGA is then sprayed onto the metal form holding the first polymer layer. The stent is sintered again to create a tri-layered structure consisting of metal-polymer type I-polymer type II. The process could be repeated with the same or additional types of polymers to build a coating of desired thickness and desired mechanical properties. Between any two layers, drug such as rapamycin or Taxol or other anti-restenotic could be dry powder coated onto any given polymer layer or the metal base stent itself.

### Example 3.

A metal, such as stainless steel, base stent is etched to a vanishingly small size while being supported on a Teflon or similarly lubricious rod. The outside diameter of the rod is slightly smaller than the inside diameter of the stent and serves to support the etched metal stent and mask the inside (luminal) surface. The mask should limit the amount of material deposited on this surface. The stent can be coated as in example 2 to achieve the desired mechanical and coating properties. Furthermore, a second drug can be deposited in any desired layer to achieve a desired elution profile.

### Example 4.

An alternative to example 3 is removal of the mask for the luminal surface of the stent. Both the stent surfaces are coated with drug(s) and polymer. The stent is supported by its own mechanical strength on the stent fixture. A single or multiple drugs (Paclitaxel or Picrolimus, for example) can be deposited in any layer of the polymer coating or through the thickness of the polymer coat.

### Example 5.

Polymer sheets created by spraying individual polymer layers on a flat surface are welded to together using compressed gas, gas or supercritical gas. A sheet of polymer is cut to a width that slightly exceeds the circumference of a finished stent. The sheet is then folded around a lubricious rod (graphite, Teflon or similar material) with an outside diameter equal to the inside diameter of a finished stent. The specific diameter is determined by the specific stent application. This object is then exposed to a gas, compressed gas or supercritical gas that can sinter the seam together forming a continuous cylinder of polymer.

The foregoing is illustrative of the present invention, and is not to be construed as limiting thereof. While embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A laminate coronary stent comprising:
a. a stent framework;
b. a plurality of layers on said stent framework to form said laminate coronary stent, where at least one of said layers comprises a bioabsorbable polymer; and
c. a layer of active agent in crystalline form and an extracellular matrix comprising an interlocking mesh of fibrous proteins and glycosaminoglycans (GAGs).

2. The laminate coronary stent of claim 1, wherein said plurality of layers comprises a plurality of bioabsorbable polymer layers deposited on said stent framework, wherein at least two of said plurality of bioabsorbable polymer layers comprise different bioabsorbable polymers having different mechanical properties.

3. The laminate coronary stent of claim 1 or 2, including a layer of at least one active agent in crystalline form.

4. The laminate coronary stent of claim 2 or 3, wherein the stent framework includes a plurality of struts, each strut having a luminal, abluminal and soluble surfaces, and wherein said plurality of bioabsorbable polymer layers are deposited on a luminal, abluminal, and soluble surfaces.

5. The laminate coronary stent of any one of claims 2 to 4, wherein each of said plurality of bioabsorbable polymer layers is sintered.

6. The laminate coronary stent of claim 3, wherein said plurality of bioabsorbable polymer layers and said layer of at least one active agent in crystalline form, form a coating on said stent framework, said coating consisting essentially of said plurality of bioabsorbable polymer layers and said layer of at least one active agent in crystalline form.

7. The laminate coronary stent of any one of claims 2 to 5, wherein said stent framework is composed of a bioabsorbable polymer or a conductive polymer.

8. The laminate coronary stent of any one of claims 1 to 5, wherein said stent framework is composed of biocompatible-non-bioabsorbable material.

9. The laminate coronary stent of any one of claims 1 to 5, wherein said stent framework is composed of stainless steel.

10. The laminate coronary stent of any one of claims 1 to 5, wherein said bioabsorbable polymer is selected from PGA poly(glycolide), LPLA poly(1-lactide), DLPLA poly(dl-lactide), PCL poly(e caprolactone), PDQ poly(dioxlane), PGA-TMC, 85115 DLPLG p(dl-lactide-co-glycolide), 75/25 DLPLG, 65/35 DLPLG, 50/50 DLPLG, TMC polytrimethylcarbonate), p(CPP:SA) ply(1,3-bis-p (carboxyphenoxy)propane-co-sebacic acid).

11. The laminate coronary stent of any one of claims 2 to 5, including 4, 10, 20, 50, and 100 or more of said plurality of bioabsorbable polymer layers.

12. The laminate coronary stent of claim 3, wherein said active agent comprises a macrolide immunosuppressive (limus) drugs.

13. The laminate coronary stent of claim 12, wherein said macrolide immunosuppressive drug comprises one or more of rapamycin, 40-0-(2-Hydroxyethyl)rapamycin (everolimus), 40-0-Benzyl-rapamycin, 40-0-(4'-Hydroxymethyl)benzyl-rapamycin, 40-0-[4'-(1,2-Dihydroxyethyl)]benzyl-rapamycin, 40-0-Allyl-rapamycin, 40-0-[3'-(2,2-Dimethyl-1,3-dioxolan-4(S)-yl)-prop-2'-en-1 '-yl]-rapamycin, (2':E,4'S)-40-0-(4',5'-Dihydroxypent-2'-en-1'-yl)-rapamycin, 40-0-(2-Hydroxy)ethoxycar-bonylmethyl-rapamycin, 40-0-(3-Hydroxy)propyl-rapamycin, 40-0-(6- Hydroxy)hexyl-rapamycin, 40-0-[2-(2-Hydroxy)ethoxy]ethyl-rapamycin, 40-0-[(3S)-2,2-Dimethyldioxolan-3-yl]methyl-rapamycin, 40-0-[(2S)-2,3-Dihydroxyprop-1-yl]-rapamycin, 40-0-(2- Acetoxy)ethyl-rapamycin, 40-0-(2-Nicotinoyloxy)ethyl-rapamycin, 40-0-[2-(N- Morpholino)acetoxy]ethyl-rapamycin, 40-0-(2-N-Imidazolylacetoxy)ethyl-rapamycin, 40-0-[2-(N Methyl-N'-piperazinyl)acetoxy]ethyl-rapamycin, 39-0-Desmethyl-39,40-0,0-ethylene-rapamycin, (26R)-26-Dihydro-40-0-(2-hydroxy)ethyl-rapamycin, 28-0-Methyl-rapamycin, 40-0-(2-Aminoethyl)-rapamycin, 40-0-(2-Acetaminoethyl)-rapamycin, 40 -0-(2-Nicotinamidoethyl) rapamycin, 40-0-(2-(N-Methyl-imidazo-2'-ylcarbethoxamido)ethyl)-rapamycin, 40-0-(2- Ethoxycarbonylaminoethyl)-rapamycin, 40-0-(2-Tolylsulfonamidoethyl)-rapamycin, 40-0-[2-(4',5' Dicarboethoxy-1',2',3'-triazol-1'-yl)-ethyl]-rapamycin, 42-Epi-tetrazolyl)rapamycin (tacrolimus), and 42-[3-hydroxy-2-(hydroxymethyl)-2-methylpropanoate]rapamycin (temsirolimus).
